# EUROPÄISCHE PATENTSCHRIFT

(11) **EP 0 680 729 B1**
(45) Veröffentlichungstag und Bekanntmachung des Hinweises auf die Patenterteilung: **29.10.1997**
(21) Anmeldenummer: 95101527.0
(22) Anmeldetag: 04.02.1995
(51) Int. Cl.: A61B 17/068

(54) **Pistole zum Setzen chirurgischer Klammern**
Pistol type surgical clip applying device
Appareil pour mettre en place des clips chirurgicaux, du type ayant une poignée pistolet

(30) Priorität: 05.05.1994 DE 4415891
(43) Veröffentlichungstag der Anmeldung: 08.11.1995
(73) Patentinhaber: WILO-Medizintechnik Lothar Wilberg GmbH, D-74424 Bühlertann (DE)
(72) Erfinder: Pistl, Gerald, D-09577 Braunsdorf (DE); Böhme, Petra, D-09130 Chemnitz (DE)
(74) Vertreter: Seerig, Dieter

(56) Entgegenhaltungen:
- WO-A-93/14701

## Beschreibung

Die Erfindung betrifft eine Pistole zum Setzen chirurgischer Klammern nach dem Oberbegriff des Anspruches.

Zur Zeit kommen aus hygienischen Gründen nur Einwegpistolen zum Setzen chirurgischer Klammern zum Einsatz. Das führt zu hohen Kosten.
Aus der DE 4303544 A1 ist ein Nachlademagazin bekannt, das am distalen Ende des endoskopischen Schaftes angesetzt wird, um aus dem Nachlademagazin Klammern in das Magazin der Pistole zu überführen. Mit dem Nachlademagazin wird die Einwegpistole für die Zeit des chirurgischen Eingriffes durch das Nachladen der Klammern im Einsatz behalten. Von Nachteil ist, daß ein komplizierter Nachlademechanismus notwendig ist, der besondere Fingerfertigkeit zum Nachladen erfordert und mechanische Störungen, z. B. Verklemmen der Klammern bei ihrer Überführung, sowie Defekte des Nachlademagazines und der Zangenschließeinrichtung nicht ausschließt. Nach Beendigung des chirurgischen Eingriffes wird die Pistole entsorgt. Ein Sterilisieren der Pistole ist praktisch nicht gegeben, da die Pistole aus mehreren Einzelteilen besteht, die einzeln desinfiziert und gereinigt werden müßten, wobei für das Auseinanderlegen und Zusammenbauen der Pistole spezielle Werkzeuge und Kenntnisse notwendig sind und Einzelteile, wie Federn vorliegen, die nur sehr schwer gereinigt werden können.

Der Erfindung liegt die Aufgabe zugrunde, eine Pistole zum Setzen chirurgischer Klammern eingangs genannter Art zu schaffen, die sich ohne Hilfsmittel demontieren und zusammenbauen läßt sowie aus wenigen, einfachen Einzelteilen besteht, die leicht zu desinfizieren und zu reinigen sowie im montierten Zustand zu sterilisieren sind.

Erfindungsgemäß wird die Aufgabe durch die im kennzeichnenden Teil des Anspruches angegebenen Merkmale gelöst.
Ein besonderer Vorteil der Erfindung liegt darin, daß die Pistole bis auf das auswechselbare Klammermagazin in wenige Einzelteile auseinandergenommen werden kann, um die Einzelteile zu desinfizieren und zu reinigen. Für das medizinische Personal ist es sehr einfach, die sterilisierte Klammerpistole unter Hinzufügen eines neuen und sterilen Klammermagazines für eine Operation vorzubereiten. Durch diesen Wiedereinsatz werden die Kosten für den chirurgischen Eingriff und für die Abfallmenge in großem Umfang verringert. Ein weiterer Vorteil ist, daß die Zangen- und Klammermagazinverriegelung gleichzeitig bedienbar sind.

Die Erfindung wird nachfolgend anhand eines Ausführungsbeispieles näher erläutert. Die dazugehörigen Zeichnungen zeigen
- Fig. 1: Gesamtansicht der Pistole
- Fig. 2: Seitenansicht des endoskopischen Schaftes mit Klammermagazin
- Fig. 3: Draufsicht des endoskopischen Schaftes mit Klammermagazin
- Fig. 4: Einzelansicht des Klammermagazines
- Fig. 5: Querschnitt A-A nach Fig. 2
- Fig. 6: Längsschnitt des Schaftes mit Klammermagazin und
- Fig. 7: Einzelheiten der Zangenschließeinrichtung

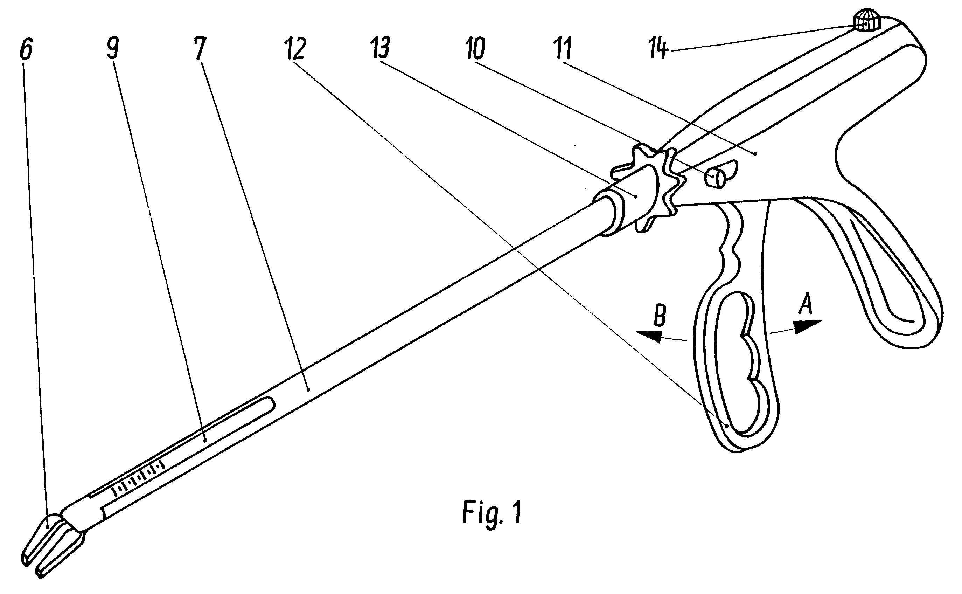

Fig. 1 zeigt die Gesamtansicht einer Pistole zum Setzen chirurgischer Klammern. Der Haltegriff 11 des endoskopischen Schaftes 7, an dessen distalen Ende ein Klammermagazin 9 und eine Zange 6 angeordnet sind, weist einen Bediengriff 12 auf. Wird der Bediengriff 12 in Richtung A bewegt, wird die Zange 6 geschlossen; eine Klammer 3 wird gesetzt. Wird der Knopf 10 gedrückt und der Bediengriff 12 in Richtung B bewegt, werden die Zangen- und Klammermagazinverriegelung geöffnet. Über die Rast-Dreh-Verstellung kann nicht nur die Zange 6 kreisförmig verstellt werden, sondern auch der Schaft 7 vom Haltegriff 11 gelöst werden. Der Haltegriff 11 ist für die Desinfektion und Reinigung mit einem Spülanschluß 14 ausgerüstet.

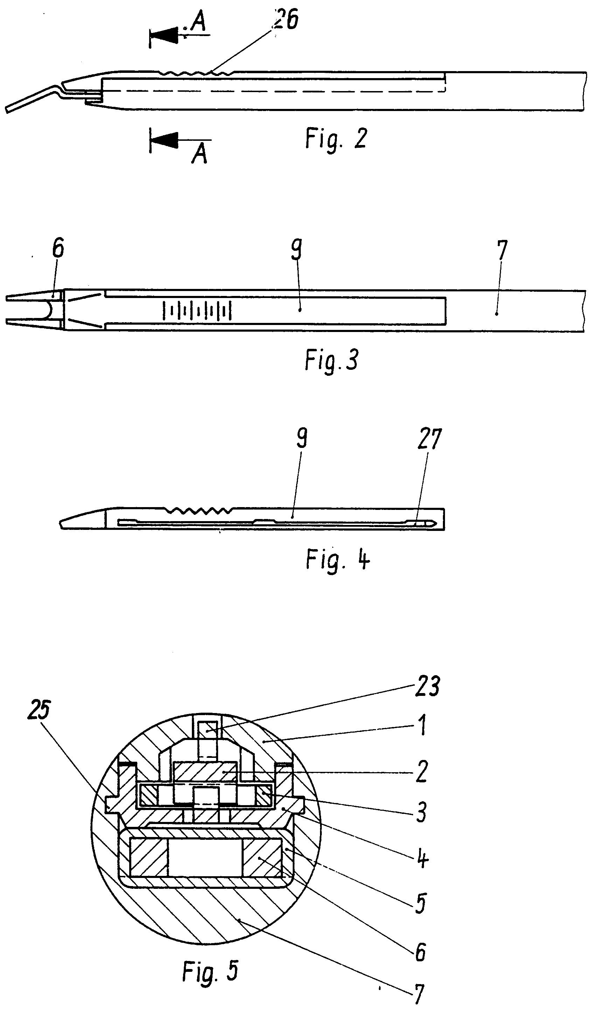

Fig. 2 zeigt den vorderen Teil des endoskopischen Schaftes 7 mit dem über Griffelement 26 eingeschobenen Klammermagazin 9 und Zange 6. Fig. 3 verdeutlicht das eingeschobene Klammermagazin 9 in den endoskopischen Schaft 7. In Figur 4 ist das Klammermagazin 9 als Einzelteil dargestellt. Dabei ist der seitliche Steg 27, welcher in seiner Breite abgesetzt ist, zu erkennen. Mit diesem Steg 27 ist das Klammermagazin 9 über das Magazinunterteil 4 in den beiden Nuten 25 im Schaft 7 geführt. In Fig. 5 ist das in die Nuten 25 eingeschobene Klammermagazin 9, das aus dem Magazinoberteil 1 besteht, welches fest mit dem Magazinunterteil 4 verbunden ist, dargestellt. Auf dem Magazinunterteil 4 ist der Vereinzelungsschieber 2 für die Klammern 3 und eine Federnase 23 zu sehen. Die Federnase 23 stützt sich gegen den Vereinzelungsschieber 2 ab. Unterhalb des Klammermagazines 9 ist die Zange 6 und die Zangenschließeinrichtung 5 eingelegt.

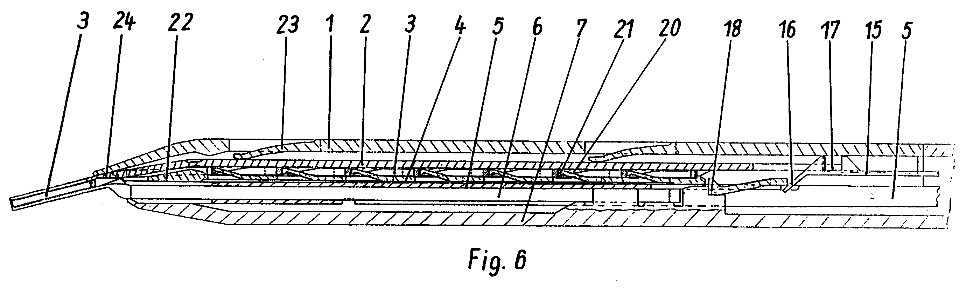

Fig. 6 zeigt das distale Ende des endoskopischen Schaftes 7 mit dem Klammermagazin 9 und der Zange 6 mit der Zangenschließeinrichtung 5 im Längsschnitt. Das Klammermagazin 9 wird über eine Klinke 18 im Schaft 7 arretiert. Die Schubstange 15 wird über eine nicht dargestellte Plattfeder bis zu einem Anschlag nach oben gedrückt. Beim Einschieben des Klammermagazines 9 in den Schaft 7 wird das Anschlußstück 17 nach oben gedrückt und über die Auflaufschräge 16 die Schubstange 15 bis zum Einrasten nach unten gedrückt. Der Hub des Anschlußstückes 17 wird in axialer Richtung über nicht dargestellte Anschläge begrenzt. Somit ist das Klammermagazin 9 funktionsfähig im Schaft 7 eingesetzt. Der Transport- und Vereinzelungsvorgang der Klammern 3 erfolgt über Bewegungen des Vereinzelungsschiebers 2 mit an diesem befestigten Rastnasen 20 und am Magazinunterteil 4 befestigten Federnasen 21. Jede einzelne Klammer 3 wird im Klammermagazin 9 von einer Federnase 21 an einer Bewegung in patientenferne Richtung gehindert. In patientenzugewandter Richtung wird durch eine Führungskulisse 22 die zufällige Bewegung der Klammern 3 aus dem Klammermagazin 9 verhindert und zusätzlich eine Verriegelung der Klammerpistole durch Einrastung der Schiebernase 24 in die Führungskulisse 22 bei leerem Klammermagazin 9 realisiert. Für den Ladevorgang einer Klammer 3 wird der Vereinzelungsschieber 2 um nicht ganz die zweifache Klammerteilung im Klammermagazin 9 in patientenentfernte Richtung bewegt. Durch die Anlaufschrägen an den Rastnasen 20 wird der Vereinzelungsschieber 2 gegen die Federnasen 23 oben angehoben und rastet patientenfern hinter alle Klammern 3 erneut ein. Zuletzt rastet die Schiebernase 24 hinter die patientennächste Klammer 3 ein. Durch die Bewegung des Vereinzelungsschiebers in Patientenrichtung wird der Abstand zwischen den beiden patientennahen Klammern 3 vergrößert. Die vorderste Klammer 3 wird in die Zange 6 geschoben. Alle weiteren Klammern 3 werden im Klammermagazin 9 um eine Teilung in Richtung Patient transportiert.

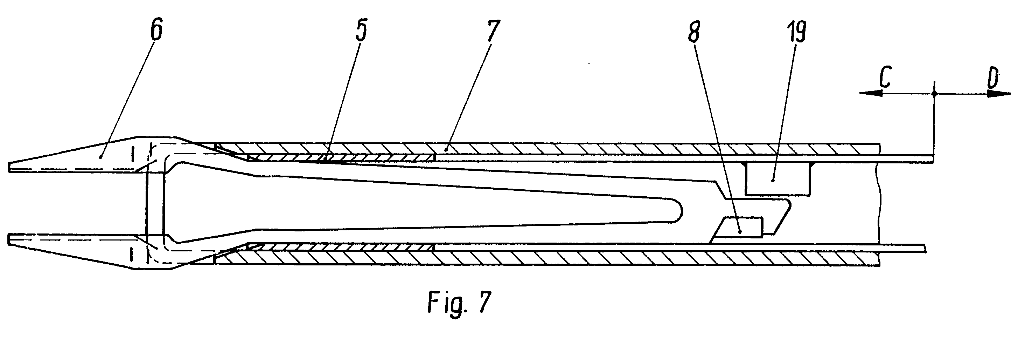

Fig. 7 verdeutlicht den einfachen Aufbau der Zange 6 mit der Zangenschließeinrichtung 5. Die Zange 6 liegt an einem Anschlag 8 an und wird über ein Füllstück 19 an der Zangenschließeinrichtung 5 in ihrer Lage gehalten.
Wird die Zangenschließeinrichtung 5 in Richtung C aus der Grundstellung bewegt, wird die Zange 6 geschlossen. Bei Bewegung aus der Grundstellung in Richtung D wird die Zangenverriegelung über das Füllstück 19 geöffnet. Gleichzeitig wird mit dieser Bewegung der Zangenschließeinrichtung 5, über je eine Auflaufschräge an der Zangenschließeinrichtung 5 und der Klinke 18, die Klinke 18 angehoben; das Klammermagazin 9 wird entriegelt.

### Aufstellung der verwendeten Bezugszeichen

- 1: = Magazinoberteil
- 2: = Vereinzelungsschieber
- 3: = Klammer
- 4: = Magazinunterteil
- 5: = Zangenschließeinrichtung
- 6: = Zange
- 7: = Schaft
- 8: = Anschlag
- 9: = Klammermagazin
- 10: = Knopf
- 11: = Haltegriff
- 12: = Bediengriff
- 13: = Rast-Dreh-Verstellung
- 14: = Spülanschluß
- 15: = Schubstange
- 16: = Auflaufschräge
- 17: = Anschlußstück
- 18: = Klinke
- 19: = Füllstück
- 20: = Rastnase
- 21: = Federnase
- 22: = Führungskulisse
- 23: = Federnase
- 24: = Schiebernase
- 25: = Nut
- 26: = Griffelement
- 27: = Steg

## Patentansprüche

1. Pistole zum Setzen chirurgischer Klammern (3), bestehend aus einem im distalen Ende eines rohrförmigen endoskopischen Schaftes (7) angeordneten Klammermagazines (9) mit einem Vereinzelungsmechanismus für die Klammern (3), einer Zange (6) mit dazugehöriger Zangenschließeinrichtung (5) zum Setzen der Klammern (3), wobei Vereinzelungsmechanismus und Zangenschließeinrichtung (5) über einen Bediengriff (12) am Haltegriff (11) der Pistole betätigbar sind, dadurch gekennzeichnet, daß das Klammermagazin (9) aus einem Magazinunterteil (4) und einem darauf fest angeordneten Magazinoberteil (1) besteht, das Klammermagazin (9) über Führungselemente in den Schaft (7) einschiebbar ist, im Klammermagazin (9) der Vereinzelungsmechanismus mit den Klammern (3) enthalten ist, unterhalb des eingeführten Klammermagazines (9) die Zange (6) mit der Zangenschließeinrichtung (5) angeordnet ist, wobei die Zange (6) auswechselbar ist, das Klammermagazin (9) an seiner dem Griff zugewandten Seite zum Einführen einer Schubstange (15) für einen Vereinzelungsschieber (2) und zum Verankern des Klammermagazines (9) im Schaft (7) offen ist, der Vereinzelungsschieber (2) ein Anschlußstück (17) aufweist, in das die Schubstange (15) einrastbar angeordnet ist, und eine federnde Klinke (18) zur Arretierung des Klammermagazines (9) im Schaft (7) angebracht ist, wobei die Klinke mit einer Auflaufschräge an einer Auflaufschräge der Zangenschließeinrichtung (5) in Wirkverbindung steht.

## Claims

1. Pistol for applying surgical clips (3), comprising a clip cartridge (9) arrangedin the distal end of a tubular endoscopic shaft (7), with a separating mechanism for the clips (3), forceps (6) with forceps closing device (5) for applying the clips (3), whereby the separating mechanism and forceps closing device (5) can be actuated using an operating grip (12) on the holding grip (11) of the pistol, characterized by the clip cartridge (9) comprising a cartridge lower section (4) and a cartridge upper section (1) fixed on it, the clip cartridge (9) being introducable by means of guide elements in the shaft (7), the separating mechanism with the clips (3) being contained in the clip cartridge (9), the forceps (6) with the forceps closing device (5) being arranged below the introduced clip cartridge (9), whereby the forceps (6) are interchangeable, the clip cartridge (9) is open on the side facing the grip for introducing a push rod (15) for a separating slide (2) and for fastening the clip magazine (9) in the shaft (7), the separating slide (2) having a connection piece (17) in which the clip-on push rod (15) is arranged, and a sprung catch (18) for locking the clip cartridge (9) in the shaft (7), the catch (18) with a contact bevel being in operating relation to a contact bevel of the forceps closing device (5).

## Revendications

1. Pistolet pour mettre en place des clips chirurgicaux (3), composé d'une réserve de clips (9) placée à l'extrémité distale d'une tige endoscopique tubulaire (7) et dotée d'un mécanisme de séparation des clips (3), d'une pince (6) avec un dispositif de fermeture de pince correspondant (5) permettant de mettre en place les clips (3), le mécanisme de séparation et le dispositif de fermeture de pince (5) peuvent être actionnés au moyen d'une poignée de commande (12) située sur la poignée de maintien (11) du pistolet et sont caractérisés par les particularités suivantes : la réserve de clips (9) est composée d'une partie inférieure (4) et d'une partie supérieure (1) fixée au dessus, la réserve de clips (9) peut être insérée dans la tige (7) par des éléments de guidage, le mécanisme de séparation est contenu dans la réserve avec les clips (3), la pince (6) est disposée avec le dispositif de fermeture (5) sous la réserve de clips (9) introduite, la pince (6) est échangeable, la réserve de clips (9) est ouverte sur le côté de la poignée pour introduire une barre poussoir (15) prévue pour un poussoir séparateur (2) et pour ancrer la réserve de clips (9) dans la tige (7), le poussoir séparateur (2) dispose d'un raccord (17), dans lequel la tige poussoir (15) est enclenchée, une poignée à ressort (18) permettant de bloquer la réserve de clips (9) est en place dans la tige (7), la poignée (18) est en rapport d'effet d'une inclinaison du dispositif de fermeture de pince (5) à une autre.
